(19) Europäisches Patentamt
European Patent Office
Office européen des brevets

(11) **EP 3 132 511 B1**

(12) # EUROPEAN PATENT SPECIFICATION

(45) Date of publication and mention
of the grant of the patent:
**24.02.2021 Bulletin 2021/08**

(21) Application number: **15717211.5**

(22) Date of filing: **09.04.2015**

(51) Int Cl.:
*H01S 5/024* *(2006.01)*   *H01S 5/068* *(2006.01)*

(86) International application number:
**PCT/GB2015/051077**

(87) International publication number:
**WO 2015/159049 (22.10.2015 Gazette 2015/42)**

(54) **WAVELENGTH CONTROL OF LASER DIODES**

WELLENLÄNGENSTEUERUNG VON LASERDIODEN

COMMANDE DE LONGUEUR D'ONDE DE DIODES LASER

(84) Designated Contracting States:
**AL AT BE BG CH CY CZ DE DK EE ES FI FR GR
HR HU IE IS IT LI LT LU LV MC MK MT NL NO PL
PT RO RS SE SI SK SM TR**

(30) Priority: **14.04.2014 GB 201406664**

(43) Date of publication of application:
**22.02.2017 Bulletin 2017/08**

(73) Proprietor: **Cranfield University
Cranfield, Bedfordshire MK43 0AL (GB)**

(72) Inventors:
• **HODGKINSON, Elizabeth Jane
Cranfield
Bedfordshire MK43 0AL (GB)**
• **TATAM, Ralph Peter
Cranfield
Bedfordshire MK43 0AL (GB)**
• **ASMARI, Abdullah Shah
Knowlhill, Milton Keynes, Buckinghamshire
MK5 8PG (GB)**

(74) Representative: **Haseltine Lake Kempner LLP
138 Cheapside
London EC2V 6BJ (GB)**

(56) References cited:
EP-A2- 0 618 653     US-A- 4 683 573
US-A1- 2004 202 210

• **DAVID GACIO ET AL: "Effects of the Junction
Temperature on the Dynamic Resistance of White
LEDs", IEEE TRANSACTIONS ON INDUSTRY
APPLICATIONS, IEEE SERVICE CENTER,
PISCATAWAY, NJ, US, vol. 49, no. 2, 1 March 2013
(2013-03-01), pages 750-760, XP011497427, ISSN:
0093-9994, DOI: 10.1109/TIA.2013.2243092**
• **FENG M X ET AL: "Thermal characterization of
GaN-based laser diodes by forward-voltage
method", JOURNAL OF APPLIED PHYSICS,
AMERICAN INSTITUTE OF PHYSICS, US, vol. 111,
no. 9, 1 May 2012 (2012-05-01), pages
94513-94513, XP012159760, ISSN: 0021-8979,
DOI: 10.1063/1.4716003 [retrieved on 2012-05-15]**
• **ASMARI A ET AL: "Wavelength stabilisation of a
DFB laser diode using measurement of junction
voltage", PROCEEDINGS OF SPIE, S P I E -
INTERNATIONAL SOCIETY FOR OPTICAL
ENGINEERING, US, vol. 9135, 1 May 2014
(2014-05-01), pages 91351A-91351A,
XP060030296, ISSN: 0277-786X, DOI:
10.1117/12.2052449 ISBN: 978-1-62841-577-3**

**Description**

FIELD OF THE INVENTION

This invention generally relates to a method of controlling emission wavelength of a light emitting device, and a system for wavelength control of a light emitting device, for example for wavelength stabilisation of a laser diode.

BACKGROUND TO THE INVENTION

Laser diode temperature is a function of ambient temperature, junction heating and thermal design of the laser diode package. Both laser diode threshold current and emission wavelength are functions of temperature. Laser diode temperature stability is of paramount importance in applications such as telecommunications and spectroscopy. High spectral resolution demands the use of singlemode operation and a wavelength-selective element. Conventional lasers used in these applications are typically distributed feedback (DFB) laser diodes, in which the wavelength-selective elements is a grating embedded along or above the active region, or vertical cavity surface emitting lasers (VCSELs), in which the wavelength-selective element is formed from interference layers that lie above and below the active region.
LEDs are often used in lower resolution, non-dispersive spectroscopy. For this technique, two or more broadband spectroscopic channels are defined using narrowband filters, one corresponding to a region of high optical absorption by the target gas (the measurement channel) and the second corresponding to a region of minimal absorption (the reference channel). The reference channel compensates for changes in the emission intensity of the broadband source. Any spectral difference in the source emission, i.e. that affects the measurement and reference channels disproportionately, will be wrongly interpreted as positive or negative optical absorption by the target species and lead to errors in the results. However, it is known that temperature changes to the LEDs can cause a distortion of the spectrum and thereby cause exactly this problem, whether in the UV, visible or infra-red regions of the spectrum. Temperature control of LEDs used in non-dispersive spectroscopy is therefore an important contributor to the limit of detection.
Laser diode operating temperature is controlled conventionally using a thermoelectric cooler (TEC) such as a Peltier element. The temperature of the laser diode is sensed with a thermistor at a short distance from the active region .The temperature precision of this technique is typically 0.01 °K. However this technique suffers from reduced accuracy as it senses the temperature of the thermistor not the laser. There is a temperature gradient between the thermistor and the laser, caused by chip heating. Despite the use of good thermal design and a case, a change in ambient temperature may cause a change to the thermal gradient, resulting in a systematic error in the emission wavelength when the ambient temperature (temperature external to the laser package) is varied. A potential alternative technique is to use the voltage drop across the laser diode to measure the temperature of the junction. This technique has been widely used in diode based thermometers for several decades. Thermometers using the junction voltage of a diode can sense temperature in the range of 1 K-400K with sensitivity of 1mK.
The laser diode central wavelength also varies with the injection current. There are two factors at work. (i) Increased heating, following an increase in injection current, causes thermal expansion of the wavelength selective element, resulting in a longer wavelength emission. This junction heating is due to non-radiative processes (where the efficiency of the laser diode in converting electrical energy into light is less than unity) as well as radiative reabsorption of light by the laser diode gain chip. (ii) Changes in injection current cause a change in refractive index of the laser diode cavity. The result is that the laser diode emission wavelength increases with increasing injection current.
Thus, the field of light emitting device control continues to provide a need for improved wavelength control.
For use in understanding the present invention, the following disclosures are referred to:

- J. H. Jeong, K. C. Kim, J. L. Lee, H. J. Kim, and I. K. Han. "Junction temperature measurement of InAs quantum-dot laser diodes by utilizing voltage-temperature method", IEEE Photonics Technology Letters 20 (16), 1354 - 1356, 2008.
- H.Y. Ryu, K. H. Ha, J. H. Chae, O. H. Nam, and Y. J. Park. "Measurement of junction temperature in GaN-based laser diodes using voltage-temperature characteristics", Applied Physics Letters 87 (9), 093506, 2005.
- Y. Xi, T. Gessman, J. Xi, J. K .Kim, J. M. Shah, E. F. Schubert, A.J,Fischer, M. H. Crawford, K. H. A. Bogart and A. A. Allerman. "Junction temperature in ultraviolet light-emitting diodes", Japanese Journal of Applied Physics 44 (10), 7260-7266, 2005.
- J. M. Smith. "Temperature compensation for gas detection" patent :WO/2009/019467
- K. Uehara and K. Katakura. "New method of frequency stabilization of semiconductor lasers", Japanese Journal of Applied Physics 27 (2), 244-246, 1988.

US4683572 (Albanese) discloses an apparatus which stabilizes the temperature, and thereby the output wavelength, of an injection laser. The laser output wavelength stabilization described therein corresponds to a method in accordance with the preamble of claim 1. EP0618653 (Shiozawa) discloses a frequency stabilization method of a semiconductor laser, a frequency-stabilized light source and a semiconductor laser module.

US2004/202210 (Thornton) discloses a control system architecture that allows a semiconductor laser to be stabilized with respect to critical parameters, such as output power and/or emission wavelength, with a reduced cost with respect to the components required to implement control, while simultaneously maintaining or increasing precision of the control function.

"Effects of the Junction Temperature on the Dynamic Resistance of White LEDs" (David Gacio et al.; IEEE TRANSACTIONS ON INDUSTRY APPLICATIONS, VOL. 49, NO. 2,MARCH 2013, pages 750-760, XP011497427) discloses dealing with the thermal characteristics of the I-V curve of GaN-based white light-emitting diodes (LEDs), focused on the variations of the dynamic resistance.

SUMMARY

According to a first aspect of the present invention, there is provided a method of controlling emission wavelength of a light emitting device, the method comprising: providing an amplitude modulated current through the device to maintain light emission of the device; during said light emission, determining a dynamic resistance indication of series resistance of the device, the series resistance comprising ohmic resistance of the device, and measuring a forward voltage of the device during said light emission, the forward voltage being across an impedance comprising impedance of an active region of the device and the series resistance; determining an indicator of active region voltage of the device on the basis of the measured forward voltage and the determined indicator of series resistance; and controlling a temperature of the device on the basis of the determined indicator of active region voltage, wherein the determining the dynamic resistance indication of series resistance comprises: extracting an AC component of forward voltage of the device while providing the amplitude modulated current; determining the dynamic resistance indication on the basis of the extracted AC component and the modulation amplitude of the amplitude modulated current, to thereby obtain the dynamic resistance indication of series resistance.

For an embodiment, an active region voltage may be termed a voltage across an active region of the device.

The active region voltage may comprise the voltage resulting from the active region, and for example might be termed the junction voltage for an active device comprising a pn junction. The series resistance may comprise the effective ohmic resistance of the device in an equivalent circuit, comprising not only the resistance of ohmic contacts but also the ohmic resistance of the active region of the device, such that the device behaves as though it has a small resistance in series with the active region, although elements of the series resistance may be co-located with the active region. (Since such behaviour may occur in an embodiment, the subject resistance is preferably termed 'series' resistance). The forward voltage may comprise the voltage that is measured across the device, which comprises the active region voltage as well as the series resistance multiplied by the injection current.

The series resistance may comprise inherent (intrinsic) resistance of the device and/or comprise resistance of ohmic contacts of the device.

Advantageously, wavelength (e.g., central, average or peak wavelength) of light emission from the device such as a laser diode may thus be accurately set and/or stabilised (when preferably driven at substantially, e.g., exactly, constant current, which may be referred to as an injection current in embodiments), even where an internal and/or external temperature of the light emitting device varies. In an embodiment, the active region voltage indication effectively allows the temperature of the active region (e.g., p-n semiconductor junction (preferably, a depletion region thereof), or quantum dot) of the device, rather than the temperature of the device as a whole, to be controlled to thereby set and/or stabilise the emission wavelength. Such temperature control may compensate for the effect on wavelength of heating of the active region of the device by injection current and/or for the effect on wavelength of ambient temperature changes. In embodiments, the light emitting device may be a quantum cascade laser (such a laser may not strictly be described as a diode) or an interband cascade laser, or broadband (LED-like) versions thereof.

For example, the determining the indication of series resistance comprises: providing an amplitude modulated current through the device to maintain light emission at a frequency f; extracting an AC (abbreviated from the well-known term 'alternating current') component (or component having a frequency that may be referred to as a high frequency; the AC and/or high frequency component preferably a first harmonic of the modulation frequency f) of forward voltage of the device while providing the amplitude modulated current; detecting a DC (abbreviated from the well-known term 'direct current') component (or component at a frequency less than f, preferably much less than f) of forward voltage of the device while providing the amplitude modulated current; determining the series resistance indication on the basis of the extracted AC component and the known amplitude of the applied AC current. Such amplitude modulation may be sinusoidal or may have any other pulsed, ramping etc.. The extracted AC component generally corresponds to, e.g., is at the same frequency as, the amplitude modulation of the current. Preferably, the modulation depth is less than 5%, preferably less than 3%, more preferably less than 1% or 0.5% of the DC amplitude of the current.

Such an embodiment may comprise inputting the forward voltage to a phase-sensitive detector to perform said extracting the AC component of forward voltage. Such a detector may be a lock-in amplifier.

There may further be provided the method, wherein the indication of series resistance is a voltage (for example, the product of current through the device and the series resistance) and: the measuring the forward voltage of the device comprises detecting a DC forward voltage of the device; the indicator of active region voltage is determined on the basis of the difference between the detected DC forward voltage and the voltage indication of series resistance. (The voltage indication of series resistance may need to be scaled). The detected DC forward voltage may be the maximum size of direct current signal detectable within the forward voltage. The detected DC forward voltage may be obtained by filtering out any AC component from the forward voltage.

There may further be provided the method, comprising: comparing the measured forward voltage and the indication of the series resistance; comparing a result of the comparison to a predetermined value, to thereby generate an error signal; performing said controlling the temperature on the basis of the error signal. The comparing the measured forward voltage and the indication of the series resistance may obtain the difference between the forward voltage and the voltage product of the current and series resistance. The predetermined value is preferably an established forward voltage setpoint or threshold.

There may further be provided the method, wherein a maximum wavelength drift of the device, when the ambient temperature of the device varies between 15 and 35 degrees Celsius, is less than +-0.03pm/degree Celsius, preferably less than +-0.02pm/degree Celsius. (This is achievable for example using an InP ridged waveguide laser and operating wavelength of 1650nm). The wavelength drift may be considered as an ambient temperature coefficient of standard deviation of emission wavelength (preferably central and/or peak) of the laser diode. Any said standard deviation is preferably measured over a fixed period, e.g., 1 hour, during which the DC component of the current through the device is substantially constant.

There may further be provided the method, wherein a standard deviation of emission wavelength (preferably central and/or peak) of the device is less than +-0.7pm, preferably less than +-0.5 pm or +-0.3pm, at an ambient temperature of 20 degrees Celsius. Again, any said standard deviation is preferably measured over a fixed period, e.g., 1 hour, during which the DC component of the current through the device is substantially constant.

There may further be provided the method, wherein a maximum variation of standard deviation of emission (preferably central and/or peak) wavelength of the device is less than +-0.6pm, preferably less than +-0.4pm, when ambient temperature of the device is varied between 15 and 35 degrees Celsius.

There may further be provided the method, wherein the light emitting device comprises a light emitting diode or laser, preferably a distributed feedback laser and/or comprising a distributed Bragg reflector. For example, the device may be an UV LED (Ultraviolet Light Emitting Diode), an InP and/or GaN laser, and/or a VCSEL (Vertical Cavity Surface Emitting Laser).

According to another aspect of the invention, there is provided a system for wavelength control of a light emitting device, the system comprising: a current driver to provide an amplitude modulated current through the device to maintain light emission of the device; a thermoelectric element for controllable heating and cooling of the device; a DC voltage detector to detect a DC component of forward voltage of the device; a phase-sensitive detector to extract an AC component of the forward voltage; a scaling circuit to scale the extracted AC component based on a modulation amplitude of the amplitude modulated current; a comparator to compare the detected DC component to the extracted AC component, to thereby indicate an active region voltage of the device; and a temperature controller configured to control the thermoelectric element on the basis of the indication of active region voltage.

The thermoelectric element may for example be a Peltier element.

The current provided through the device is preferably to bias the device with a DC injection current, while further comprising an AC component to allow the phase-sensitive detection to occur.

There may further be provided the system, wherein the temperature controller comprises a proportional integral and derivative controller configured to control the thermoelectric element on the basis of the indication of active region voltage.

There may further be provided the system, wherein the light emitting device comprises a light emitting diode or laser, preferably a distributed feedback laser and/or comprising a distributed Bragg reflector.

There may be provided a spectrometer comprising the system, the spectrometer preferably for detecting one or more gases having absorption spectra that fall within a range of wavelengths emitted by the device, for example for detecting methane.

Preferred embodiments are defined in the appended dependent claims.

BRIEF DESCRIPTION OF THE DRAWINGS

For a better understanding of the invention and to show how the same may be carried into effect, reference will now be made, by way of example, to the accompanying drawings, in which:

Fig. 1 shows laser diode centre wavelength stability with ambient temperature under junction voltage control;

Fig. 2 shows a 3D model of a DFB laser diode in a butterfly package;

Fig. 3 shows an investigation of conventional method of laser diode wavelength stabilisation;

Fig. 4 shows laser diode wavelength drift with ambient temperature while conventionally controlled to a thermistor temperature of 15 °C, at constant injection current of 150mA;

Fig. 5 shows a "See-saw" model of conventional laser diode control, using a thermistor set point;

Fig. 6 shows laser diode long term wavelength stability at constant ambient temperature of 30 °C , thermistor temperature of 15 °C and constant injection current of 150mA;

Fig. 7 shows a flow chart showing forward voltage based control of Peltier current;

Fig. 8 shows a schematic of forward voltage method to stabilise laser diode wavelength;

Fig. 9 shows laser diode wavelength stability with variable ambient temperature under forward voltage control;

Fig. 10 shows a laser diode long term wavelength stability test using forward voltage based control;

Fig. 11 shows an example laser diode small signal model;

Fig. 12 shows laser diode series resistance measurement operating under thermistor based control at 10 °C;

Fig. 13 shows a V-I plot of the laser diode operating under thermistor based control at different temperatures;

Fig. 14 shows a flow chart for laser diode wavelength stabilisation using junction voltage;

Fig. 15 shows configuration of junction voltage method for stabilising the wavelength of the laser diode;

Fig. 16 shows a methane 3f signal from a gas cell used for wavelength measurement;

Fig. 17 shows an expanded section of Fig. 16, showing a linear region around the line centre. The equation of a best fit straight line through the experimental points is also given;

Fig. 18 shows long term wavelength stability for the laser diode using junction voltage based control;

Fig. 19 shows laser diode wavelength deviation from the methane line centre (1650.96 nm) under junction voltage control, at ambient temperatures of 15°C and 35°C;

Fig. 20(a - c) show Labview code used for laser diode wavelength stability using forward voltage based control.

## DETAILED DESCRIPTION OF PREFERRED EMBODIMENTS

Generally, embodiments provide wavelength control of laser diodes via measurement of junction voltage, also termed active region voltage of the laser diode. Specifically, wavelength stabilisation may be achieved in an embodiment by using the voltage drop across a laser diode to measure the temperature of the active medium instead of a thermistor. Advantageously, the junction voltage rather than the forward voltage is used. Since the series resistance of these devices imposes a small but significant error when measuring the forward voltage, by measuring this resistance and compensating for it, the junction voltage alone is measured, the accuracy of the measurement is improved significantly and/or the emission wavelength is stabilised, preferably with no measurable systematic error when the ambient temperature is varied in the range 15-35°C. The junction temperature may be measured more accurately, and/or the effects of ambient temperature may be compensated.

To aid understanding, we now consider firstly the V-I relationship of a laser diode.

The injection current and laser diode junction voltage can be represented by Shockly equation

$$I_f = I_s exp\left(\frac{eV_j}{\eta kT}\right) \qquad (1)$$

Where $I_f$ and $I_s$ are forward and saturation current of the laser diode respectively. $V_j$ is the junction voltage of the laser diode, $e$ is the electronic charge, $k$ is the Boltzmann's constant, and $T$ is the thermodynamic temperature in degrees Kelvin. $\eta$ is the ideality factor and is approximately equal to 1 for a laser diode operated above the threshold.

The voltage of the laser diode can be derived from the Shockly equation

$$V_j = ln\frac{I_f}{I_s}\frac{\eta kT}{e} \qquad (2)$$

When $\frac{\eta kT}{e} \ll V_j$, which is also called the thermal voltage then

$$\frac{dV_j}{d_T} = \frac{d}{dT}\left[\frac{\eta kT}{e} \, ln\frac{I_f}{I_s}\right] \qquad (3)$$

Where

$$I_s = \left[T^3 exp\left(\frac{-E_g}{kT}\right)\right] T^{\frac{\gamma}{2}} \qquad (4)$$

$E_g$ is the band gap energy measured in volts and $\gamma$ is a constant The $T^{\frac{\gamma}{2}}$ term is close to unity, and the saturation current can be approximated as

$$I_s = T^3 exp\left(\frac{-E_g}{kT}\right) C \qquad (5)$$

where C is a constant. Substituting equation (5) in equation (3),

$$\frac{dV_j}{d_T} = \frac{d}{dT}\left[\frac{\eta kT}{e} \, ln\left(\frac{I_f}{C}\right) - \frac{3lnT\eta kT}{e} + \frac{\eta E_g}{e}\right] \qquad (6)$$

The above relationship gives the temperature dependence of the laser diode junction voltage. The forward voltage $V_f$ of the laser can be described as

$$V_f = V_j + I_f R_s \qquad (7)$$

$V_f$ and $R_s$ are the voltage measured across the terminals of the laser diode and the series resistance of the laser diode. The derivative of the junction voltage in terms of forward voltage can be written as

$$\frac{dV_j}{dT} = \frac{d}{dT}\left[\frac{\eta kT}{e} \, ln\left(\frac{I_f}{C}\right) - \frac{3lnT\eta kT}{e} + \frac{\eta E_g}{e}\right] - \frac{d}{dT}(I_f R_s) \qquad (8)$$

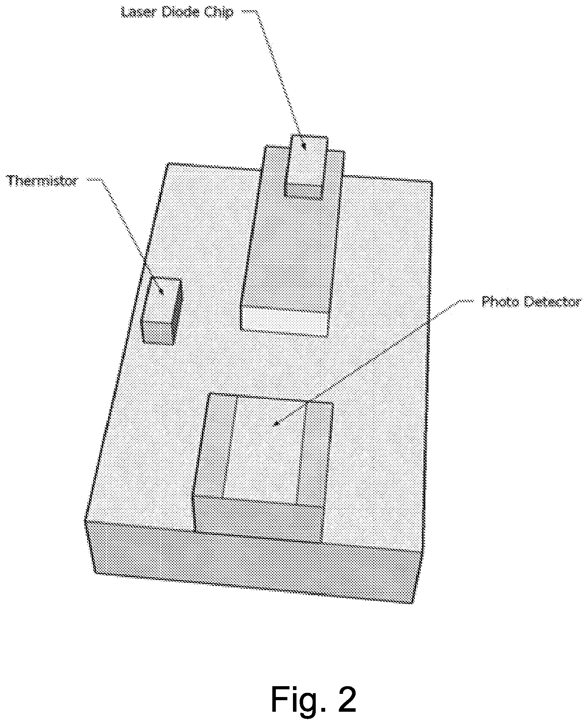

Considering now laser diode wavelength stability using conventional thermistor based control, it is noted that, in a conventional laser diode temperature control system, the temperature of the laser diode chip is sensed with a thermistor placed at a distance as shown in Fig. 2. The thermistor gives a temperature measurement of its immediate surroundings.

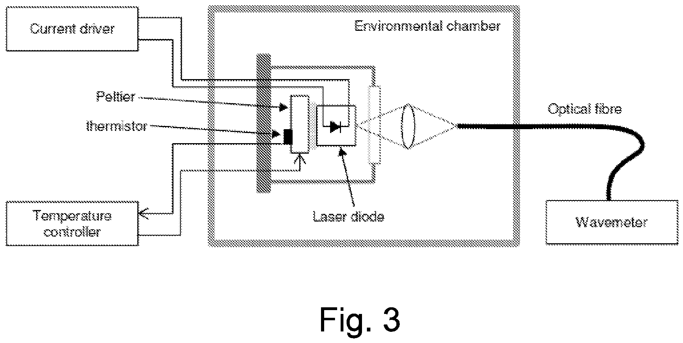

Regarding equipment and experimental configuration, an InP ridged waveguide laser with a wavelength of 1650nm (HHI TO8 MTE Module) was investigated for wavelength stability using conventional thermistor-based control. The thermistor inside the laser diode package was connected to a temperature controller (Thorlabs TED200), which in turn was connected to the Peltier element in the package in order to control the thermistor temperature. The injection current to the laser diode was supplied using a current driver (Thorlabs LDC200). An environmental chamber was used to maintain constant external ambient temperature to within $\pm$ 1°C, as shown in Fig. 3. The wavelength of the laser diode was measured with a wavelength meter (Agilent AG86122B). The resolution of this wavemeter was 100 fm and its absolute accuracy was 0.3 pm. Because the wavemeter could not measure wavelengths longer than 1650nm, for this test the laser diode was operated at 1649nm.

The laser diode was operated above threshold at different ambient temperatures, placed in an environmental chamber in the range of 15-35°C. The laser diode injection current was kept constant at 150mA $\pm$ 0.5mA and the thermistor temperature was maintained at 15°C $\pm$ 0.01°C.

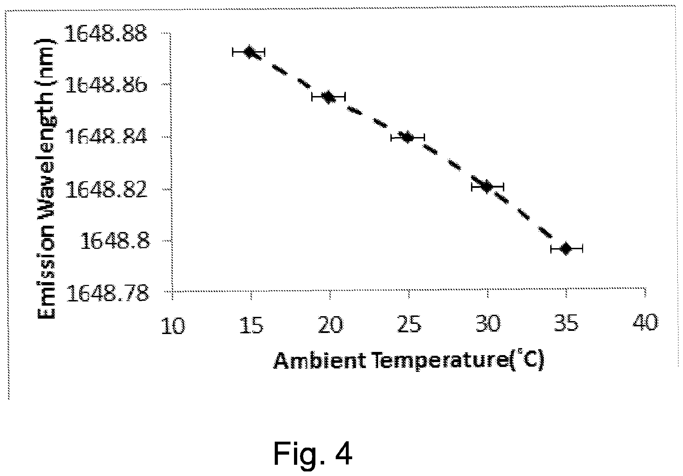

Considering the results for conventional control, Fig. 4 demonstrates the effect of variable ambient temperature on the laser diode wavelength. The ambient temperature for the laser diode was varied in 5°C steps by changing the environmental chamber temperature.

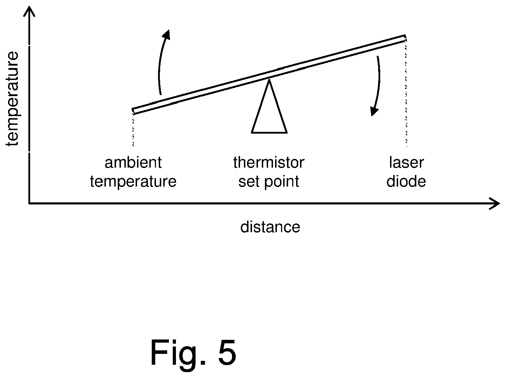

As the ambient temperature was increased from 15 °C to 35 °C, the laser diode emission wavelength drifted to shorter wavelengths, despite having constant injection current and thermistor temperature. This is consistent with the hypothesis that the thermistor was not measuring the actual temperature of the laser diode and as the ambient temperature was increased, the temperature gradient between the chip and the thermistor was affected. Therefore, the laser diode chip was further cooled down. Fig. 5 illustrates this principle with a simple "see-saw" model.

Thus, the laser diode wavelength drifted towards shorter wavelengths with a total wavelength shift of 77pm over the temperature range of 15-35°C. The change in laser diode wavelength with ambient temperature was -3.8pm$\pm$0.5pm /°C at a constant thermistor temperature.

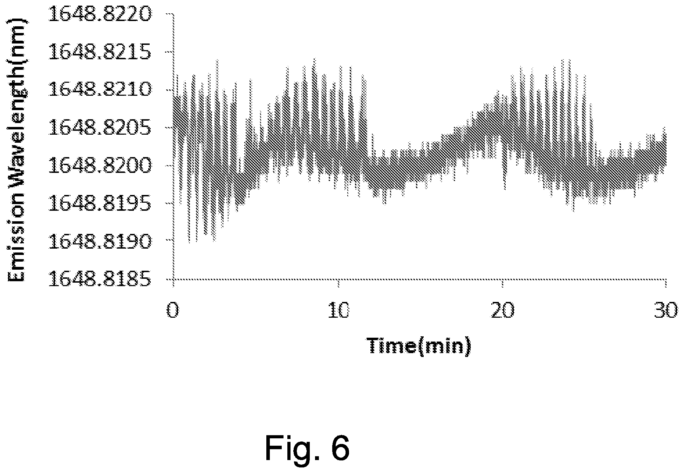

The conventional thermistor control method was also tested for long term laser wavelength stability. The injection current to the laser diode was kept constant at 150mA $\pm$ 0.5mA and the thermistor temperature was controlled to 15°C $\pm$0.01°C. The ambient temperature was set to 30 °C and the wavelength monitored for 30min. Fig. 6 shows the resulting long term wavelength stability. The laser diode wavelength remained stable over time with a standard deviation of $\sigma=\pm$0.4pm over a time of 30 minutes.

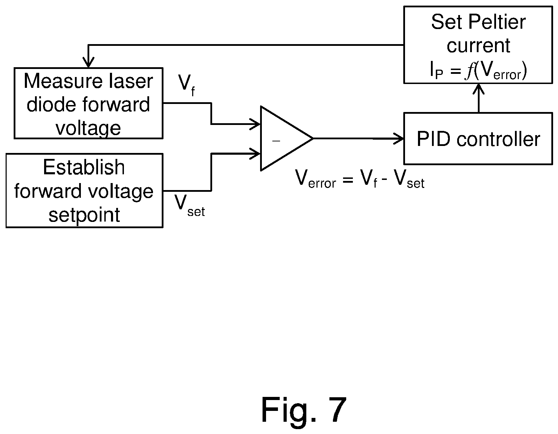

Turning now to an assessment of laser diode wavelength stability using forward voltage based control, the laser forward voltage was used as a temperature sensor to stabilise the wavelength of the laser diode. The equipment and experimental setup involved the same DFB laser used in the above described investigation of conventional method of laser diode wavelength stabilisation. The laser was investigated for wavelength stability using a forward voltage method. The laser diode was driven using the same current driver (ThorLabs LDC200) and its emission was monitored using the same wave meter (Agilent AG86122B) as described in the above discussion of the investigation of conventional method of laser diode wavelength stabilisation. The laser diode was again placed in an environmental chamber whose temperature could be changed in the range 15-35°C. A Labview (National Instruments) based proportional integral and derivative (PID) controller was used to implement a forward voltage based temperature controller, as shown in the flowchart in Fig. 7.

The laser diode was driven with a constant injection current of 150mA $\pm$ 0.5mA. The voltage drop measured while the thermistor was controlled to 15°C was used as setpoint voltage for the PID controller. The ambient external temperature of the environmental chamber was varied in steps of 5°C over the range 15-35°C.

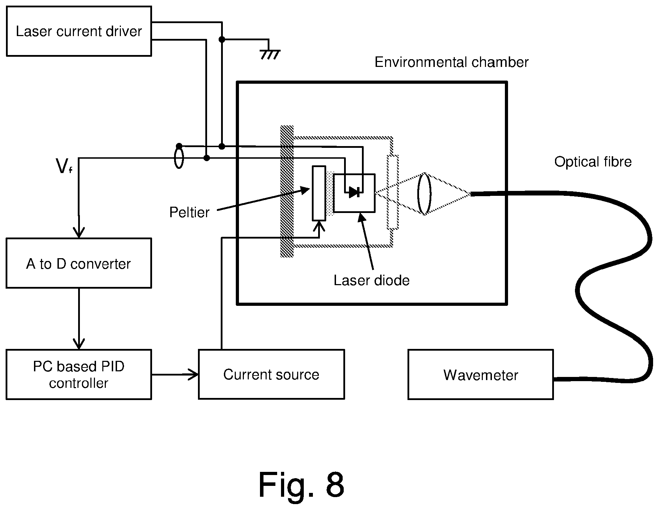

The voltage drop across the laser diode was measured and compared to the voltage setpoint. The difference between these voltages acted as an error signal, which was fed into the PID controller to stabilise the temperature of the laser diode as described in Fig. 8.

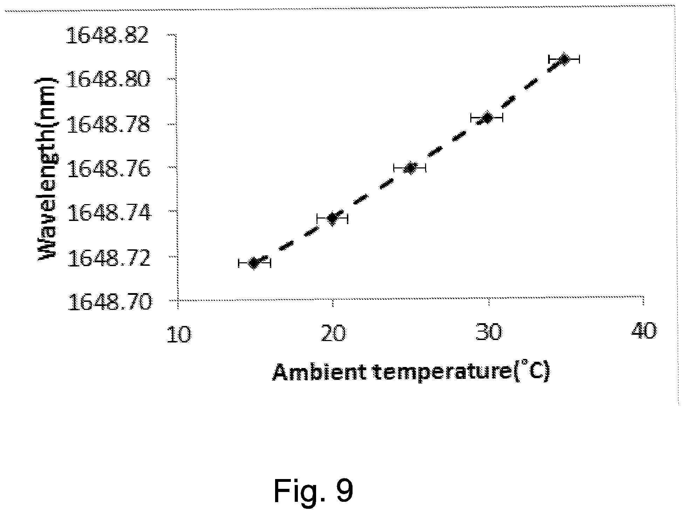

Regarding the results, Fig. 9 shows the relationship between ambient temperature and laser diode emission wavelength under forward voltage control. The laser diode wavelength was found to drift towards longer wavelengths at higher ambient temperatures, in contrast to the drift observed using the conventional method, where the wavelength became shorter as described in section (3.2). It was found that this technique suffered from a systematic error of 4.5 pm / °C with changes in ambient temperature, as can be observed in the graph in Fig. 9.

Fig. 9 shows that the laser diode wavelength drifted towards longer wavelengths at higher ambient temperatures, indicating that the forward voltage is not the only important parameter for measurement of laser junction temperature. The total wavelength drift was 90 pm over the range 15-35°C. The performance of the forward voltage method in stabilising the wavelength of the laser diode with variable ambient temperature is therefore comparable to that of conventional thermistor based control.

The forward voltage technique was also tested for long-term wavelength stability. The laser diode was kept in an environmental chamber at a constant temperature of 30 °C $\pm$ 1 °C. The laser diode chip was biased with an injection current of 150mA $\pm$ 0.5mA and the wavelength was measured with the wavemeter (described above in relation to the conventional method of laser diode wavelength stabilisation) over a period of 30 minutes.

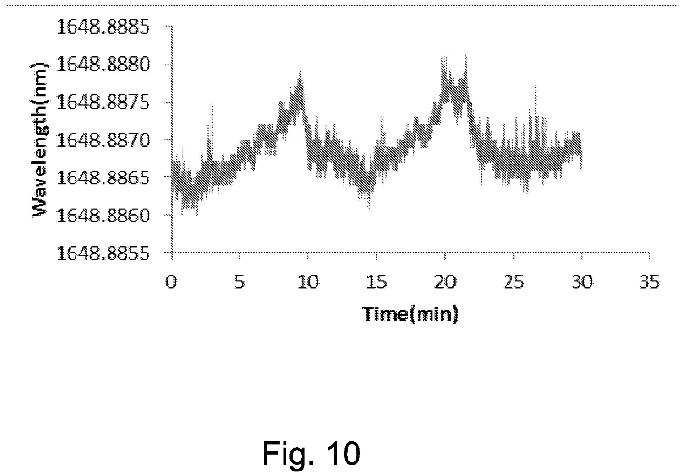

Fig. 10 shows the long term wavelength stability of the laser diode at constant temperature and injection current. The wavelength stability was $\pm$0.32pm (1$\sigma$), which again is comparable to conventional thermistor based control.

The long-term wavelength stability and systematic error with ambient temperature changes were comparable for temperature control using the forward voltage method and using the thermistor. However, tuneable diode laser spectroscopy of gases such as methane, where absorption line the half width half maximum is 50pm (100% methane concentration at atmospheric pressure), requires a more stable laser diode wavelength when faced with varying ambient temperatures. Therefore, an improved wavelength control method is desirable.

We now consider laser diode junction voltage based control. The forward voltage is the voltage measured across the anode and cathode of the laser diode. The forward voltage is the sum of junction voltage and the voltage drop across the series resistance of the laser diode, $R_s$ as described in equation (7). Both junction voltage and series resistance are temperature dependent as shown in equation (8). As described in the following, the effect of temperature on forward voltage and series resistance are investigated. The laser diode junction voltage is investigated for the purpose of stabilising the wavelength of the laser diode over long timescales and in the face of variable ambient temperature.

The laser diode series resistance can be measured as follows. From the first derivative of the Shockley equation (1) combined with equation (7), and assuming $V_f \gg V_T$, we have

$$\frac{dV_f}{dI_f} = \left(\frac{kT\eta}{q}\right) \cdot \frac{1}{I_f} + R_s \qquad (9)$$

Which can be rewritten as

$$R = R_j + R_s \qquad (10)$$

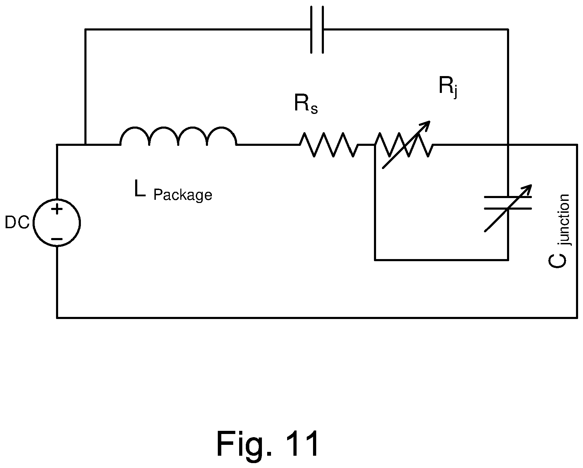

$R$ and $R_j$ are the total dynamic resistance and effective junction resistance of the laser diode respectively. $R_s$ is the additional series resistance of the laser diode caused by ohmic effects within the device. Fig. 11 shows the small signal model of a laser diode, which may be an equivalent circuit that may be used to understand how the laser diode behaves.

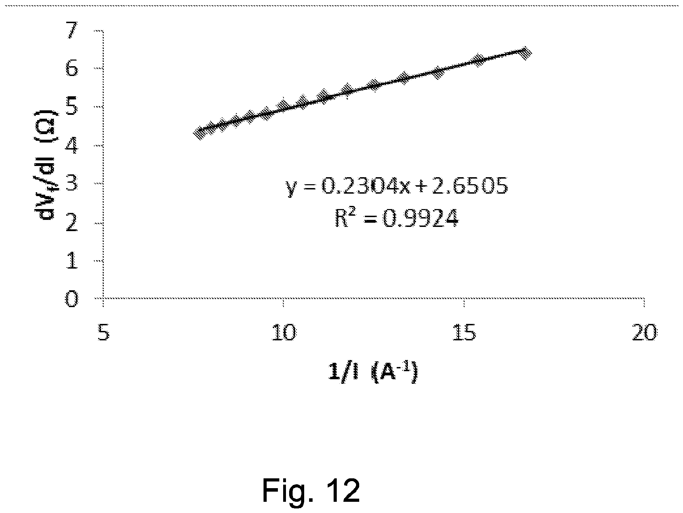

The laser diode series resistance $R_s$ increases with increasing temperature. To determine the value of $R_s$, the forward voltage of the laser diode was measured for a range of injections currents at different operating temperatures. By plotting $\frac{dV_f}{dI}$ against the injection current ($I$), the laser diode resistance can be measured as shown in Fig. 12.

Fig. 12 shows the laser diode series resistance at 10°C; the plot shows a linear relationship between $\frac{dV_f}{dI}$ and the inverse of the injection current. The y-axis intercept of this graph gives the measurement of laser diode series resistance, which increases with increasing thermistor temperature as given in Table 1. We have found that the temperature dependence of $R_s$ in equation (10) is significant, and requires compensation if the forward voltage is to be used for temperature control purposes.

Measurement of the dynamic resistance as $\frac{dV_f}{dI}$ in equation (9) gives a value whose variation with temperature is substantially influenced by that of the series resistance $R_s$, and this can therefore can be used to provide this compensation.

Table 1. The effect of temperature on the laser diode series resistance $R_s$

| Operating temperature(°C) | Laser Diode Resistance $R_s$ ($\Omega$) |
|---|---|
| 10 | 2.65 |
| 20 | 2.70 |
| 30 | 2.74 |
| 40 | 2.86 |

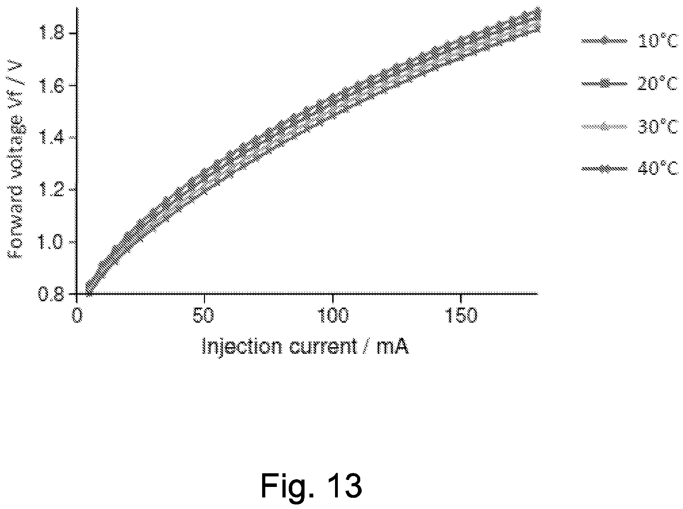

A plot of voltage versus injection current over the temperature range 10-40°C in Fig. 13 shows that with increasing temperature, the forward voltage decreases, in contrast to the series resistance of the laser diode as shown in Table 1 which increases.

The graphs in Figs. 12 and 13 confirm that there is a small additional series resistance within the laser diode, which is temperature dependent as shown in Table 1. The voltage drop due to the laser diode series resistance will contribute to the forward voltage by adding a small voltage that is dependent on both temperature and injection current. Therefore, this potentially adds a systematic error to the measurement of temperature of the laser diode using the forward voltage of the laser diode. This systematic error in temperature sensing using forward voltage can be observed in Fig. 9, where the laser diode emission has drifted towards longer wavelengths due to the increasing ambient temperature.

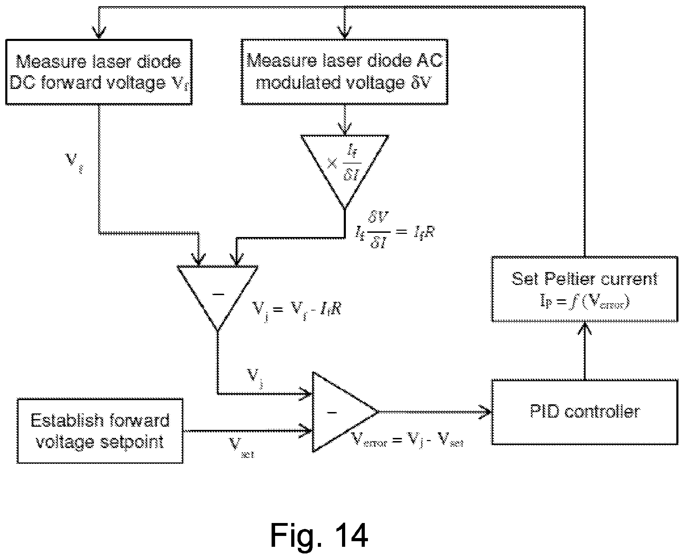

Regarding equipment and experimental setup, the flow chart in Fig. 14 shows a process of stabilising the laser diode wavelength using voltage across the active region of the device (e.g., laser diode junction voltage, the junction generally being a semiconductor pn junction) as opposed to merely forward voltage (forward voltage generally being that dropped between the anode and cathode of the device, e.g., across the impedance represented by the full equivalent circuit of Fig. 11). The laser diode dynamic resistance R was measured by modulating the laser diode using a relatively small modulation depth sinusoidal current (which may be termed an amplitude modulated current), while simultaneously applying a DC injection current above the threshold. The voltage drop across the laser diode was fed into a lock in amplifier and demodulated at 1f. The demodulated signal gave a measure of the laser dynamic resistance R as $\partial V_f/\partial I_f$ at the DC current. In an embodiment, a phase-sensitive detector in the form of a lock-in amplifier is used to extract the AC component $\delta V$ from the detected forward voltage $V_f$, the extracted signal being processed (e.g., scaled by dividing by the modulation amplitude $\delta I$ of the modulated current and/or multiplying by the DC amplitude $I_f$ of the modulated current), preferably to provide the measure of the laser resistance as $\partial V_f/\partial I_f$.

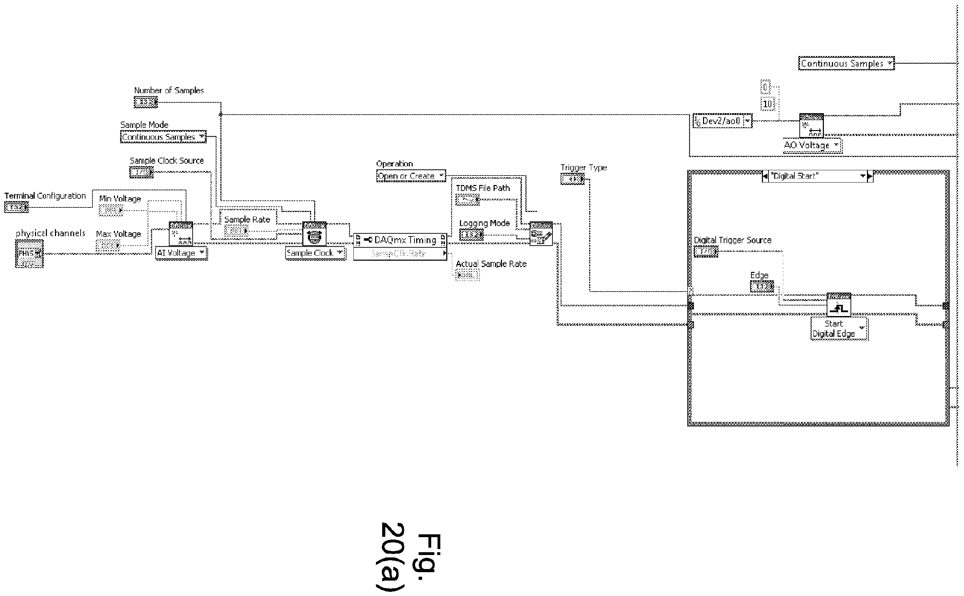

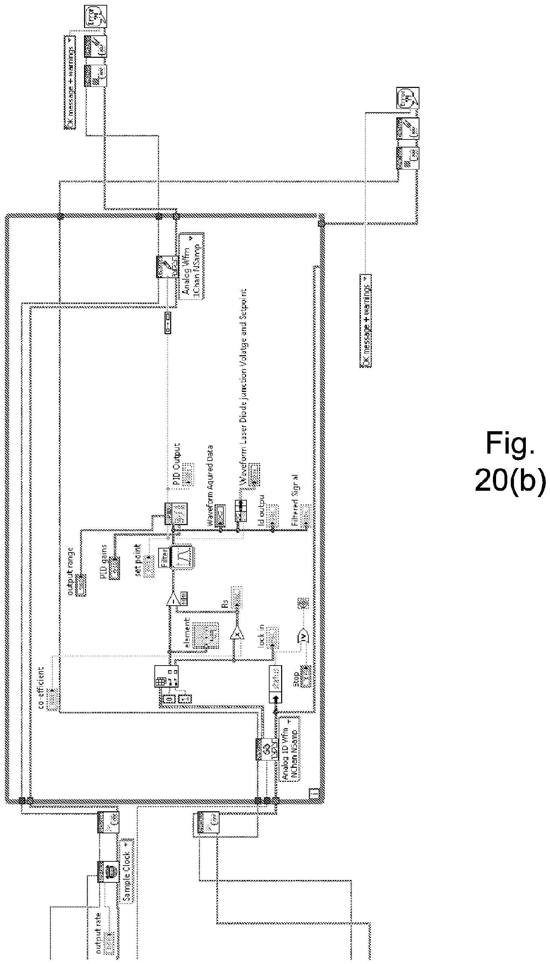

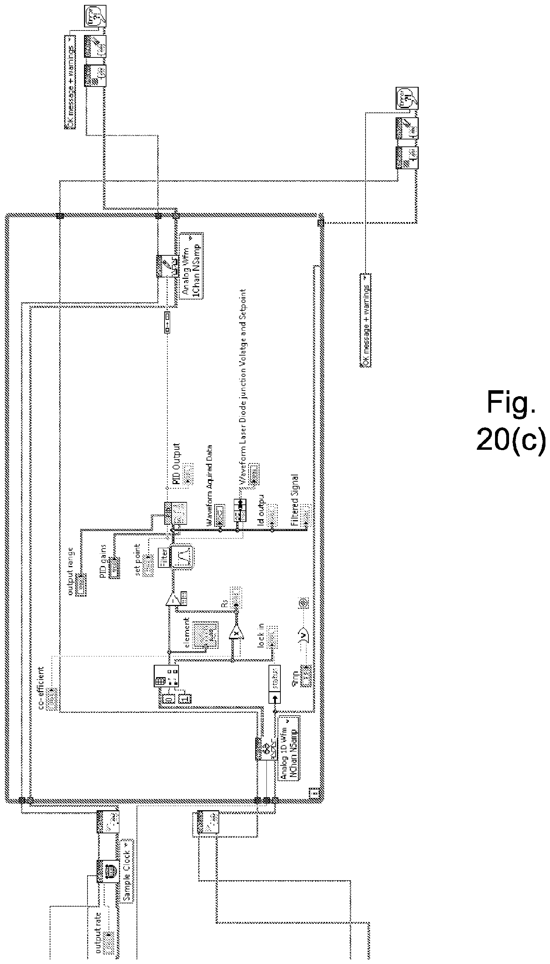

The same PID controller was used as described above in relation to the equipment and experimental setup for laser diode wavelength stability using forward voltage based control. The Labview code (Fig. 20) used for the above experimental setup for laser diode wavelength stability using forward voltage based control was modified as follows. The RMS modulated voltage was measured using a lock-in amplifier and multiplied by an empirically determined factor ($I/\delta I$ in Fig. 14) to give an indication of the additional voltage due to the laser resistance (IR). This was subtracted from the DC forward voltage to give an indicator of the active region voltage in the form of a measure of the junction voltage $V_j$. A setpoint value $V_{set}$ was subtracted from $V_j$ to provide an error signal in the PID feedback loop to the Peltier element.

In practice, the scale factors shown in Fig. 14 are used to give a suitable starting point for the voltage compensation, and are then adjusted empirically to take account of the minor effects of $R_j$ and any additional ohmic contacts.

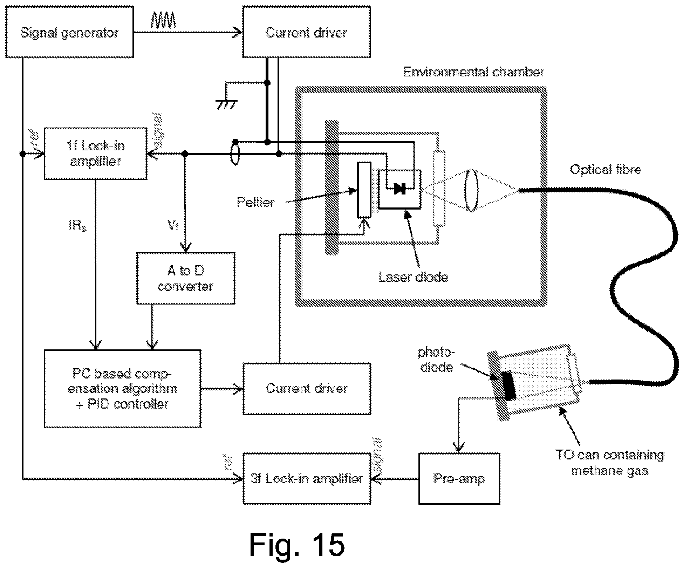

Fig. 15 shows the experimental configuration for laser diode wavelength control using the laser diode junction voltage. The laser diode was modulated at 31 kHz with a peak-to-peak amplitude of 5.2 mA. A current driver (Thorlabs LDC200) was used to bias the diode with DC injection current of 165.8 mA. A lock-in amplifier (Stanford Research SR850) was used to measure the 1f modulated forward voltage, providing a resistance measurement to compensate the forward voltage as shown in Fig. 14. The resulting junction voltage was compared to a setpoint voltage to provide an error signal to the PID. Voltages were measured using a data acquisition card (National Instruments PCI 6259); voltage compensation and the PID were all implemented in Labview. The output from the PID was sent to the modulation input of a second current driver (ThorLabs ITC510) in order to close the feedback loop to the Peltier cooler.

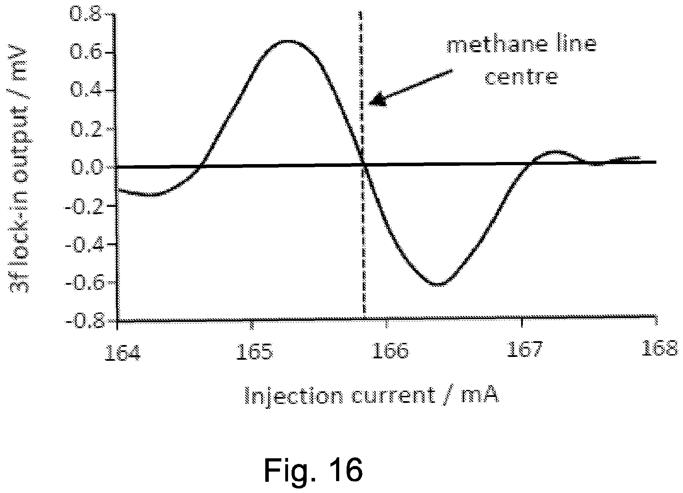

The resolution of an optical spectrum analyser was too coarse to measure the level of wavelength stability resulting from this method, and additionally it would measure the time-averaged emission corresponding to envelope of the wavelength modulation resulting from the applied AC current. Therefore, the laser diode emitted wavelength was measured with the help of a methane reference cell, an 8mm long TO can filled with methane at approximately 2.5% volume and containing an InGaAs photodiode. The signal from the photodiode pre-amplifier was demodulated at 3f using a lock-in amplifier (Stanford SRS 850) in the manner of wavelength modulation spectroscopy. The 3f signal passes through zero at the gas line centre (see Fig. 16) therefore this signal offered a convenient measure of the deviation of the emitted wavelength from the line centre at 1650.96 nm. In this technique, the deviation of the centre wavelength is measured; the small wavelength modulation means that the wavelength as constantly varying.

Considering now gas reference cell wavelength calibration, a configuration involved a laser diode driven using a sinusoidal signal with frequency 31 kHz and p-p amplitude 5.2 mA, giving a p-p wavelength modulation of 115 pm. A simultaneous DC bias was applied in the range 164 - 168 mA.

To establish a wavelength calibration, the centre wavelength of the laser diode was scanned across methane gas line while simultaneously applying the sinusoidal modulation. The detector output was then fed into the lock-in amplifier and the third harmonic was measured. The results are shown in Fig. 16. The zero crossing at an applied current of 165.8 mA of the 3f signal corresponds to the peak of the methane gas line. To make sure that the line centre has been identified, the calibration process was repeated while observing the 1f, 2f and 3f signals.

A wavelength calibration was established as follows, giving the rate of change of 3f signal with centre wavelength $\lambda$.

$$\frac{d(3f\ signal)}{d(\lambda)} = \frac{d(3f\ signal)}{d(I_f)} \times \frac{d(I_f)}{d(\lambda)} \tag{11}$$

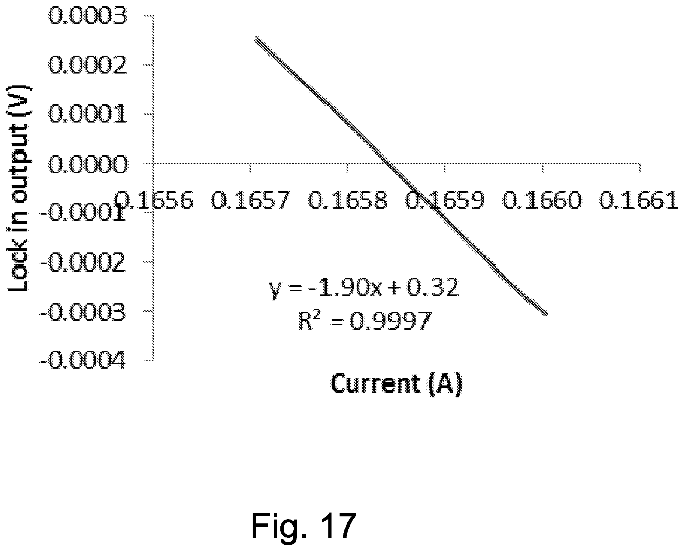

The laser diode wavelength tuning co-efficient, $d(\lambda)/d(I_f)$, was established to be 23 pm / mA by measuring the wavelength at different DC injection currents using the optical spectrum analyser. The value of $d(3f\ signal)/d(I_f)$ was established by expanding the plot in Fig. 16, giving a linear region shown in Fig. 17 below. This gave a gradient of 1.90 V /A.

By this method, the voltage output from the 3f lock-in amplifier was established to have a calibration coefficient of $d(3f\ signal)/d(\lambda) = 81\ \mu V$ / pm.

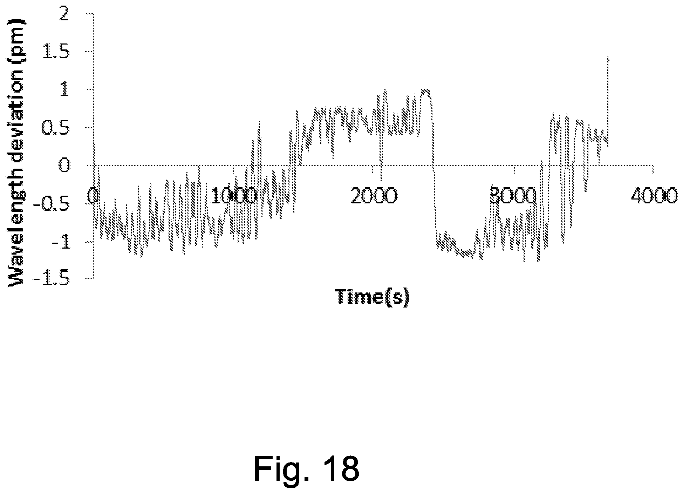

Regarding the results, the laser was operated under junction voltage control for a period of one hour at a constant ambient temperature of 20°C and a DC injection current of 165.8 mA. The lock-in amplifier 3f signal was recorded and converted into wavelength using the calibration procedure described above in relation to gas reference cell wavelength calibration. Fig. 18 shows the resulting wavelength deviation from the gas line centre over time.

The laser diode remained stable over a period of one hour with a wavelength standard deviation of $\sigma = \pm 0.7$ pm. The long-term wavelength stability of the laser diode with forward voltage was therefore comparable to that of the thermistor and forward voltage control methods.

The junction voltage control technique was also tested for wavelength stability with changes in ambient temperature in the range of 15 - 35°C.

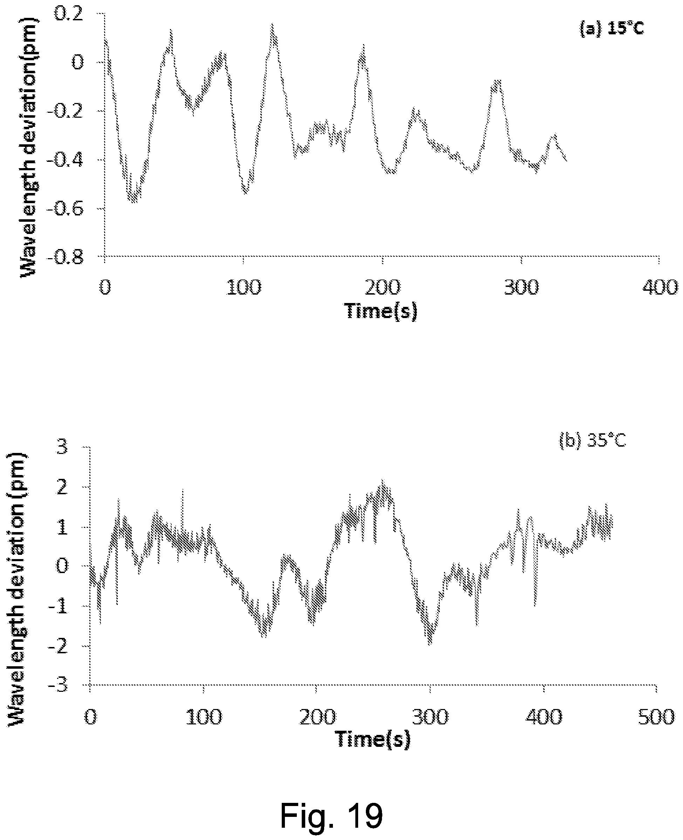

Fig. 19 shows the response of the laser diode wavelength over time and to a change in ambient temperature. In both figures, the 3f voltage output remained close to the zero crossing point, indicating the laser diode wavelength was near the centre of the methane gas line.

Measurements have shown the laser diode wavelength drift with varying ambient temperature. The laser diode wavelength remained stable with ambient temperature coefficient of $\leq 0.03$ pm/°C. The laser diode wavelength stability with ambient temperature using junction voltage is therefore 2 orders of magnitude better than that achieved using conventional thermistor control and using the forward voltage control technique.

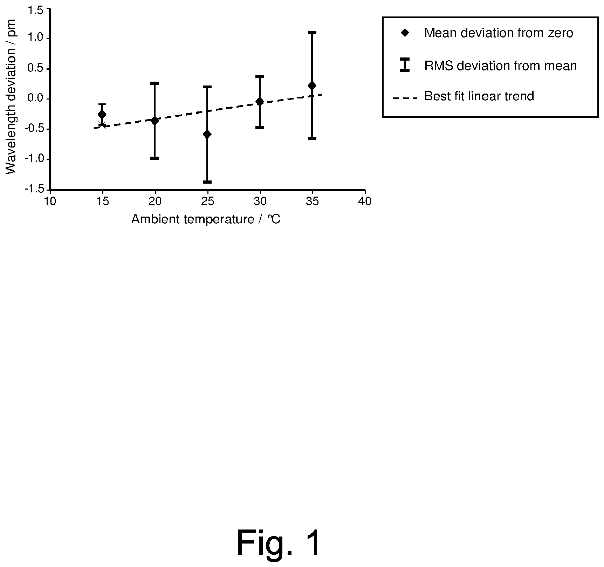

Regarding laser diode centre wavelength stability with ambient temperature under junction voltage control, we refer to Fig. 1.

In view of the above, an approximately linear relationship between DFB laser diode voltage and temperature can be used as a temperature sensor and, within a control loop, to stabilise the central wavelength of the laser diode. The laser diode forward voltage decreased approximately linearly with increasing operating temperature. In conventional laser diode temperature control, a thermistor placed at a distance is used to sense the temperature of the laser diode chip. The thermistor measures the temperature of its immediate surroundings. However, there is a temperature gradient between the chip and the thermistor, resulting in laser diode wavelength drift when the ambient temperature changes. It was experimentally demonstrated that, under conventional thermistor control, the laser diode wavelength decreased with increasing ambient temperature.

The voltage across the laser diode (forward voltage) can be used to stabilise the emission wavelength of the laser diode. The performance of the forward voltage control technique was comparable to that of conventional thermistor control. However, with variable ambient temperatures the laser diode wavelength drifted due to the temperature dependence of the laser diode series resistance. The laser diode forward voltage decreased with increasing temperature, whereas laser diode series resistance increased with increasing temperature. Therefore, when the ambient temperature was increased, the forward voltage decreased, resulting in laser diode wavelength drift towards longer wavelengths.

The laser diode series resistance was measured over a range of operating temperatures. The laser diode resistance increased with increasing temperature, in contrast to a decrease in the forward voltage. A control system was developed based on measurement of the junction voltage of the laser diode to stabilise its wavelength. The laser diode resistance was measured dynamically, by modulating the injection current with a DC bias above threshold. The laser diode was modulated at a high frequency (31 kHz) with a small amplitude to minimise the additional heat generated due to modulation. The 1f demodulated output was scaled and subtracted from the voltage drop measured across the laser diode. The resulting junction voltage was subtracted from a voltage setpoint and the result used as an error signal in a PID control loop.

Due to the limited resolution of the spectrum analyser, a methane gas line was used to check the wavelength stability of the laser diode when using junction voltage control. The centre wavelength of the laser diode was checked for long-term stability and under variable ambient temperatures. The laser diode central wavelength had a long-term stability similar to that of the forward voltage and thermistor control methods, as shown below in Table 2. However, when faced with different ambient temperatures, junction voltage control showed a mean wavelength stability 2 orders of magnitude better than that of either thermistor or forward voltage control.

Table 2 Comparison of laser diode wavelength (or frequency) control techniques

| Control technique | Long term stability (30 min) | Wavelength drift with ambient temperature |
| --- | --- | --- |
| Thermistor control | $\pm$ 0.4 pm ($\pm$ 40 MHz) | 3.8pm /°C (420 MHz / °C) |
| Junction voltage control | $\pm$ 0.6 pm ($\pm$ 70 MHz) | $\leq$ 0.03 pm / °C ($\leq$3.3 MHz / °C) |

Tuneable laser diode spectroscopy requires a very stable laser diode wavelength as the gas absorption lines are very narrow, e.g. the methane absorption at 1650.96nm has a linewidth (HWHM) of 50pm for 100% concentration. For an ambient temperature change of 20°C, conventional thermistor control would suffer a wavelength change of 76 pm, greater than the methane gas linewidth at 1650.96nm. Using junction voltage control would bring this down to a more manageable 0.6 pm, which is much smaller than the gas linewidth and similar to the level of wavelength stability over time at a fixed temperature.

The technique is applicable to various packages, e.g., the TO can - packaged DFB described here, a butterfly-packaged DFB and a VCSEL, all at the same or other wavelength, and a UV, visible or IR LED. Generally, the laser may be a distributed feedback laser wherein the active region of the laser comprises a diffraction grating. The laser may be comprise a distributed Bragg reflector, e.g., may be vertical cavity surface emitting laser. The laser may by InP- or GaN-based. The laser may be a quantum dot laser.

**Claims**

1. Method of controlling emission wavelength of a light emitting device, the method comprising:

   providing an amplitude modulated current ($I_f$) through the device to maintain light emission of the device;
   during said light emission, determining a dynamic resistance indication of series resistance (Rs) of the device, the series resistance comprising ohmic resistance of the device, and measuring a forward voltage ($V_f$) of the device during said light emission, the forward voltage being across an impedance comprising impedance of an active region of the device and the series resistance;
   determining an indicator of active region voltage ($V_j$) of the device on the basis of the measured forward voltage and the determined dynamic resistance indication of series resistance; and
   controlling a temperature of the device on the basis of the determined indicator of active region voltage,
   **characterised in that**
   the determining the dynamic resistance indication of series resistance comprises:

   extracting an AC component of forward voltage of the device while providing the amplitude modulated current;
   determining the dynamic resistance indication on the basis of the extracted AC component and the modulation amplitude of the amplitude modulated current, to thereby obtain the dynamic resistance indication of series resistance.

2. Method of claim 1, comprising inputting the forward voltage ($V_f$) to a phase-sensitive detector to perform said extracting the AC component.

3. Method of any preceding claim, wherein the dynamic resistance indication of series resistance is a voltage and:

   the measuring the forward voltage ($V_f$) of the device comprises detecting a DC forward voltage of the device;
   the indicator of active region voltage ($V_j$) is determined on the basis of the difference between the detected DC forward voltage and the voltage indication of series resistance.

4. Method of any preceding claim, comprising:

   comparing the measured forward voltage ($V_f$) and the dynamic resistance indication of the series resistance ($R_s$);
   comparing a result of the comparison to a predetermined value, to thereby generate an error signal;
   performing said controlling the temperature on the basis of the error signal.

5. Method of any preceding claim, wherein a maximum wavelength drift of the device, when the ambient temperature of the device varies between 15 and 35 degrees Celsius, is less than +-0.03pm/degree Celsius, preferably less than +-0.02pm/degree Celsius.

6. Method of any preceding claim, wherein a standard deviation of emission wavelength of the device is less than +-0.7pm, preferably less than +-0.3pm, at an ambient temperature of 20 degrees Celsius.

7. Method of any preceding claim, wherein a maximum variation of standard deviation of emission wavelength of the device is less than +-0.6pm, preferably less than +-0.4pm, when ambient temperature of the device is varied between 15 and 35 degrees Celsius.

8. Method of any preceding claim, wherein the light emitting device comprises a light emitting diode or laser, preferably a distributed feedback laser and/or comprising a distributed Bragg reflector or VCSEL.

9.  System for wavelength control of a light emitting device, the system comprising:

    a current driver configured to provide an amplitude modulated current ($I_f$) through the device to maintain light emission of the device;
    a thermoelectric element for controllable heating and cooling of the device;
    a DC voltage detector configured to detect a DC component of forward voltage ($V_f$) of the device;
    a phase-sensitive detector configured to extract an AC component of the forward voltage;
    a scaling circuit configured to scale the extracted AC component based on a modulation amplitude of the amplitude modulated current;
    a comparator configured to compare the detected DC component to the scaled extracted AC component, to thereby indicate an active region voltage ($V_j$) of the device; and
    a temperature controller configured to control the thermoelectric element on the basis of the indication of active region voltage.

10. System of claim 9, wherein the temperature controller comprises a proportional integral and derivative controller configured to control the thermoelectric element on the basis of the indication of active region voltage ($V_j$).

11. System of any one of claims 9 and 10, wherein the light emitting device comprises a light emitting diode or laser, preferably a distributed feedback laser and/or comprising a distributed Bragg reflector or VCSEL.

12. Spectrometer comprising the system of any one of claims 9 to 11.

13. Spectrometer of claim 12 for detecting one or more gases having absorption spectra that fall within a range of wavelengths emitted by the device.

14. Spectrometer of claim 12 or 13 for detecting methane.


**Patentansprüche**

1.  Verfahren zum Regulieren von Emissionswellenlänge einer lichtemittierenden Vorrichtung, das Verfahren umfassend:

    Bereitstellen eines amplitudenmodulierten Stroms ($I_f$) durch die Vorrichtung, um eine Lichtemission der Vorrichtung aufrechtzuerhalten;
    während der Lichtemission, Bestimmen einer dynamischen Widerstandsanzeige des Reihenwiderstands ($R_s$) der Vorrichtung, der Reihenwiderstand umfassend ohmschen Widerstand der Vorrichtung, und Messen einer Durchlassspannung ($V_f$) der Vorrichtung während der Lichtemission, wobei die Durchlassspannung über einer Impedanz ist, die eine Impedanz einer aktiven Region der Vorrichtung und den Reihenwiderstand umfasst;
    Bestimmen eines Indikators für die Spannung einer aktiven Region (Vj) der Vorrichtung basierend auf der gemessenen Durchlassspannung und der bestimmten dynamischen Widerstandsanzeige des Reihenwiderstands; und
    Regulieren einer Temperatur der Vorrichtung basierend auf dem bestimmten Indikator der Spannung der aktiven Region,
    **dadurch gekennzeichnet, dass** das Bestimmen der dynamischen Widerstandsanzeige des Reihenwiderstands Folgendes umfasst:

    Extrahieren einer Wechselstromkomponente von Durchlassspannung der Vorrichtung, während der amplitudenmodulierte Strom bereitgestellt wird;
    Bestimmen der dynamischen Widerstandsanzeige basierend auf der extrahierten Wechselstromkomponente und der Modulationsamplitude des amplitudenmodulierten Stroms, um dadurch die dynamische Widerstandsanzeige des Reihenwiderstands zu erlangen.

2.  Verfahren gemäß Anspruch 1, umfassend ein Eingeben der Durchlassspannung ($V_f$) in einen phasenempfindlichen Detektor, um das Extrahieren der Wechselstromkomponente auszuführen.

3.  Verfahren gemäß einem der vorherigen Ansprüche, wobei die dynamische Widerstandsanzeige des Reihenwiderstands eine Spannung ist und:

das Messen der Durchlassspannung ($V_f$) der Vorrichtung ein Erfassen einer Gleichstrom-Durchlassspannung der Vorrichtung umfasst;

die Anzeige der Spannung der aktiven Region ($V_j$) basierend auf der Differenz zwischen der erfassten Gleichstrom-Durchlassspannung und der Spannungsanzeige des Reihenwiderstands ermittelt wird.

4. Verfahren gemäß einem der vorherigen Ansprüche, umfassend:

Vergleichen der gemessenen Durchlassspannung ($V_f$) und der dynamischen Widerstandsanzeige des Reihenwiderstands ($R_s$);

Vergleichen eines Ergebnisses des Vergleichs mit einem vorbestimmten Wert, um dadurch ein Fehlersignal zu erzeugen;

Ausführen des Regulierens der Temperatur basierend auf dem Fehlersignal.

5. Verfahren gemäß einem der vorherigen Ansprüche, wobei eine maximale Wellenlängenverschiebung der Vorrichtung, wenn die Umgebungstemperatur der Vorrichtung zwischen 15 und 35 Grad Celsius variiert, weniger als +-0,03 pm/Grad Celsius, vorzugsweise weniger als +-0,02 pm/Grad Celsius ist.

6. Verfahren gemäß einem der vorherigen Ansprüche, wobei eine Standardabweichung von Emissionswellenlänge der Vorrichtung bei einer Umgebungstemperatur von 20 Grad Celsius weniger als +-0,7pm, vorzugsweise weniger als +-0,3pm, ist.

7. Verfahren gemäß einem der vorherigen Ansprüche, wobei eine maximale Variation einer Standardabweichung von Emissionswellenlänge der Vorrichtung weniger als +-0,6pm, vorzugsweise weniger als +-0,4pm, ist, wenn eine Umgebungstemperatur der Vorrichtung zwischen 15 und 35 Grad Celsius variiert wird.

8. Verfahren gemäß einem der vorherigen Ansprüche, wobei die lichtemittierende Vorrichtung eine lichtemittierende Diode oder einen Laser, vorzugsweise einen Laser mit verteilter Rückkopplung, umfasst und/oder einen verteilten Bragg-Reflektor oder VCSEL umfasst.

9. System zur Wellenlängenregulierung einer lichtemittierenden Vorrichtung, das System umfassend:

einen Stromtreiber, der konfiguriert ist, um einen amplitudenmodulierten Strom ($I_f$) durch die Vorrichtung bereitzustellen, um eine Lichtemission der Vorrichtung aufrechtzuerhalten;

ein thermoelektrisches Element für ein regulierbares Heizen und Kühlen der Vorrichtung;

einen Gleichspannungsdetektor, der konfiguriert ist, um eine Gleichstromkomponente einer Durchlassspannung ($V_f$) der Vorrichtung zu detektieren;

einen phasenempfindlichen Detektor, der konfiguriert ist, um eine Wechselstromkomponente der Durchlassspannung zu extrahieren;

eine Skalierungsschaltung, die konfiguriert ist, um die extrahierte Wechselstromkomponente basierend auf einer Modulationsamplitude des amplitudenmodulierten Stroms zu skalieren;

einen Komparator, der konfiguriert ist, um die detektierte Gleichstromkomponente mit der skalierten extrahierten Wechselstromkomponente zu vergleichen, um dadurch eine Spannung einer aktiven Region ($V_j$) der Vorrichtung anzugeben; und

einen Temperaturregler, der konfiguriert ist, um das thermoelektrische Element basierend auf der Angabe einer Spannung der aktiven Region steuert.

10. System gemäß Anspruch 9, wobei der Temperaturregler einen Proportional-Integral- und Derivativregler umfasst, der konfiguriert ist, um das thermoelektrische Element basierend auf der Angabe einer Spannung der aktiven Region ($V_j$) zu regulieren.

11. System gemäß einem der Ansprüche 9 und 10, wobei die lichtemittierende Vorrichtung eine lichtemittierende Diode oder einen Laser, vorzugsweise einen Laser mit verteilter Rückkopplung, umfasst und/oder einen verteilten Bragg-Reflektor oder VCSEL umfasst.

12. Spektrometer, umfassend das System gemäß einem der Ansprüche 9 bis 11.

13. Spektrometer gemäß Anspruch 12 zum Detektieren eines oder mehrerer Gase die Absorptionsspektren aufweisen, die in einen Bereich von Wellenlängen fallen, die von der Vorrichtung emittiert werden.

**14.** Spektrometer gemäß Anspruch 12 oder 13 zum Detektieren von Methan.


**Revendications**

**1.** Procédé de commande de la longueur d'onde d'émission d'un dispositif électroluminescent, le procédé comprenant :

la fourniture d'un courant modulé en amplitude ($I_f$) à travers le dispositif pour maintenir l'émission de lumière du dispositif ;

Pendant ladite émission de lumière, la détermination d'une indication de résistance dynamique de la résistance en série ($R_s$) du dispositif, la résistance en série comprenant la résistance ohmique du dispositif et la mesure d'une tension directe ($V_f$) du dispositif pendant ladite émission de lumière, la tension directe étant à travers une impédance comprenant l'impédance d'une région active du dispositif et la résistance en série ;

la détermination d'un indicateur de la tension de la région active ($V_i$) du dispositif sur la base de la tension directe mesurée et de l'indication de résistance dynamique déterminée de la résistance en série ; et

la commande d'une température du dispositif sur la base de l'indicateur déterminé de la tension de la région active,

**caractérisé en ce que** la détermination de l'indication de résistance dynamique de la résistance en série comprend :

l'extraction d'une composante CA de la tension directe du dispositif tout en fournissant le courant modulé en amplitude ;

la détermination de l'indication de résistance dynamique sur la base de la composante CA extraite et de l'amplitude de modulation du courant modulé en amplitude, ce qui permet d'obtenir l'indication de résistance dynamique de la résistance en série.

**2.** Procédé selon la revendication 1, comprenant l'entrée de la tension directe ($V_i$) dans un détecteur sensible à la phase pour effectuer ladite extraction de la composante CA.

**3.** Procédé selon l'une quelconque des revendications précédentes, dans lequel l'indication de résistance dynamique de la résistance en série est une tension et :

la mesure de la tension directe ($V_f$) du dispositif comprend la détection d'une tension directe CC du dispositif ;

l'indicateur de tension de la région active ($V_i$) est déterminé sur la base de la différence entre la tension directe CC détectée et l'indication de tension de la résistance en série.

**4.** Procédé selon l'une quelconque des revendications précédentes, comprenant :

la comparaison de la tension directe mesurée ($V_i$) et de l'indication de résistance dynamique de la résistance en série (Rs) ;

la comparaison d'un résultat de la comparaison à une valeur prédéfinie, ce qui permet de générer un signal d'erreur ;

la réalisation de ladite commande de la température sur la base du signal d'erreur.

**5.** Procédé selon l'une quelconque des revendications précédentes, dans lequel une dérive maximale de la longueur d'onde du dispositif, lorsque la température ambiante de l'appareil varie entre 15 et 35 degrés Celsius, est inférieure à ±0,03 pm/degré Celsius, de préférence inférieure à ±0,02 pm/degré Celsius.

**6.** Procédé selon l'une quelconque des revendications précédentes, dans lequel un écart-type de la longueur d'onde d'émission du dispositif est inférieur à ±0,7 pm, de préférence inférieur à ±0,3 pm, à une température ambiante de 20 degrés Celsius.

**7.** Procédé selon l'une quelconque des revendications précédentes, dans lequel une variation maximale de l'écart-type de la longueur d'onde d'émission de l'appareil est inférieure à ±0,06 pm, de préférence inférieure à ±0,04 pm, lorsque la température ambiante de l'appareil varie entre 15 et 35 degrés Celsius.

**8.** Procédé selon l'une quelconque des revendications précédentes, dans lequel le dispositif émettant de la lumière comprend une diode électroluminescente ou un laser, de préférence un laser à rétroaction distribuée et/ou comprenant un réflecteur Bragg distribué ou un laser à cavité verticale et à émission par la surface (VCSEL).

9. Système destiné à la commande de longueur d'onde d'un dispositif électroluminescent, le système comprenant :

un circuit d'attaque de courant configuré pour fournir un courant modulé en amplitude ($I_f$) à travers le dispositif pour maintenir l'émission de lumière du dispositif ;
un élément thermoélectrique destiné au chauffage et au refroidissement du dispositif pouvant être commandé ;
un détecteur de tension CC configuré pour détecter une composante CC de la tension directe ($V_f$) du dispositif ;
un détecteur sensible à la phase configuré pour extraire une composante CA de la tension directe ; un circuit de mise à l'échelle configuré pour mettre à l'échelle la composante CA extraite en fonction d'une amplitude de modulation du courant modulé en amplitude ;
un comparateur configuré pour comparer la composante CC détectée sur la composante CA extraite mise à l'échelle, ce qui permet d'indiquer une tension de la région active ($V_i$) du dispositif et un dispositif de commande de température configuré pour commander l'élément thermoélectrique sur la base de l'indication de tension de la région active.

10. Système selon la revendication 9, dans lequel le dispositif de commande de température comprend un dispositif de commande proportionnel intégral et dérivé configuré pour commander l'élément thermoélectrique sur la base de l'indication de la tension de la région active ($V_i$).

11. Système selon l'une quelconque des revendications 9 et 10, dans lequel le dispositif électroluminescent comprend une diode électroluminescente ou un laser, de préférence un laser à rétroaction distribuée et/ou comprenant un réflecteur Bragg distribué ou un VCSEL.

12. Spectromètre comprenant le système selon l'une quelconque des revendications 9 à 11.

13. Spectromètre selon la revendication 12 destiné à détecter un ou plusieurs gaz présentant des spectres d'absorption qui tombent à l'intérieur d'une plage de longueurs d'onde émises par le dispositif.

14. Spectromètre selon la revendication 12 ou 13 destiné à détecter du méthane.

Fig. 1

Fig. 2

Current driver

Environmental chamber

Peltier

thermistor

Laser diode

Optical fibre

Temperature controller

Wavemeter

# Fig. 3

Fig. 4

Fig. 5

Fig. 6

Fig. 7

Fig. 8

Fig. 9

Fig. 10

Fig. 11

Fig. 12

Fig. 13

Measure laser diode
DC forward voltage $V_f$

Measure laser diode AC
modulated voltage $\delta V$

$V_f$

$\times \dfrac{I_f}{\delta I}$

$I_f \dfrac{\delta V}{\delta I} = I_f R$

$-$   $V_j = V_f - I_f R$

Set Peltier current
$I_p = f(V_{error})$

$V_j$

Establish forward
voltage setpoint

$V_{set}$

$-$

$V_{error} = V_j - V_{set}$

PID controller

Fig. 14

Fig. 15

Fig. 16

Fig. 17

Fig. 18

Fig. 19

Fig. 20(a)

Fig. 20(b)

Fig. 20(c)

**EP 3 132 511 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

Patent documents cited in the description

- WO 2009019467 A
- US 4683572 A, Albanese
- EP 0618653 A, Shiozawa
- US 2004202210 A, Thornton

**Non-patent literature cited in the description**

- **J. H. JEONG ; K. C. KIM ; J. L. LEE ; H. J. KIM ; I. K. HAN.** Junction temperature measurement of InAs quantum-dot laser diodes by utilizing voltage-temperature method. *IEEE Photonics Technology Letters,* 2008, vol. 20 (16), 1354-1356
- **H.Y. RYU ; K. H. HA ; J. H. CHAE ; O. H. NAM ; Y. J. PARK.** Measurement of junction temperature in GaN-based laser diodes using voltage-temperature characteristics. *Applied Physics Letters,* 2005, vol. 87 (9), 093506
- **Y. XI ; T. GESSMAN ; J. XI ; J. K .KIM ; J. M. SHAH ; E. F. SCHUBERT ; A.J,FISCHER ; M. H. CRAWFORD ; K. H. A. BOGART ; A. A. ALLERMAN.** Junction temperature in ultraviolet light-emitting diodes. *Japanese Journal of Applied Physics,* 2005, vol. 44 (10), 7260-7266
- **K. UEHARA ; K. KATAKURA.** New method of frequency stabilization of semiconductor lasers. *Japanese Journal of Applied Physics,* 1988, vol. 27 (2), 244-246
- **DAVID GACIO et al.** Effects of the Junction Temperature on the Dynamic Resistance of White LEDs. *IEEE TRANSACTIONS ON INDUSTRY APPLICATIONS,* March 2013, vol. 49 (2), 750-760